# EUROPEAN PATENT APPLICATION

(11) **EP 3 840 076 A1**
(43) Date of publication of application: **23.06.2021**
(21) Application number: 19219160.9
(22) Date of filing: 20.12.2019
(51) Int. Cl.: H01L 51/00

(54) **ORGANIC ELECTRONIC DEVICE COMPRISING A COMPOUND OF FORMULA (1), DISPLAY DEVICE COMPRISING THE ORGANIC ELECTRONIC DEVICE AS WELL AS COMPOUNDS OF FORMULA (1) FOR USE IN ORGANIC ELECTRONIC DEVICES**

(71) Applicant: Novaled GmbH, 01099 Dresden (DE)
(72) Inventor: Uvarov, Vladimir, 01099 Dresden (DE); Heggemann, Ulrich, 01099 Dresden (DE); Willmann, Steffen, 01099 Dresden (DE)
(74) Representative: Michalski Hüttermann & Partner Patentanwälte mbB

(57) **Abstract**

The present invention relates to an organic electronic device comprising a semiconductor layer which comprises a compound of formula (1)

## Description

### Technical Field

The present invention relates to an organic electronic device comprising a compound of formula (1) and a display device comprising the organic electronic device. The invention further relates to novel compounds of formula (1) which can be of use in organic electronic devices.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of metal complexes which are also contained in the semiconductor layer.

There remains a need to improve performance of organic semiconductor materials, semiconductor layers, as well as organic electronic devices thereof, in particular to achieve improved operating voltage stability over time through improving the characteristics of the compounds comprised therein.

Additionally, there is a need to provide compounds with improved thermal properties.

### DISCLOSURE

An aspect of the present invention provides an organic electronic device comprising an anode, a cathode, at least one photoactive layer and at least one semiconductor layer, wherein the at least one semiconductor layer is arranged between the anode and the at least one photoactive layer; and wherein the at least one semiconductor layer comprises a compound of Formula (1) 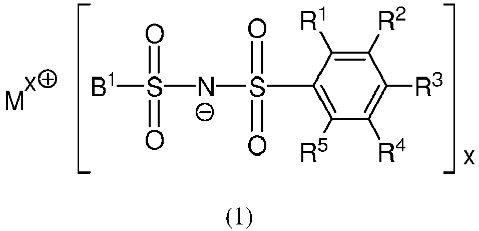 Wherein
M is a metal ion
x is the valency of M
B¹ is selected from substituted or unsubstituted C₁ to C₁₆ alkyl,
R¹ to R⁵ are independently selected from H, F, CN, halogen, substituted or unsubstituted C₁ to C₆ alkyl, substituted or unsubstituted C₆ to C₁₂ aryl, substituted or unsubstituted C₃ to C₁₂ heteroaryl,
wherein the substituents on B¹ and/or R¹ to R⁵ selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR⁶, COOR⁶, halogen, F or CN;
and where at least one of R¹ to R⁵ is selected from substituted or unsubstituted C₁ to C₆ alkyl or CN.

The negative charge in compounds of formula (1) may be delocalised partially or fully over the N(SO₂)₂ group and optionally also over the B¹ and substituted phenyl groups.

It should be noted that throughout the application and the claims any Bⁿ ,Rⁿ etc. always refer to the same moieties, unless otherwise noted.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an iso-propyl group, a butyl group, an iso-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms

In the present specification, the single bond refers to a direct bond.

In the context of the present invention, "different" means that the compounds do not have an identical chemical structure.

The term "free of', "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms anode and anode electrode are used synonymously.

The terms cathode and cathode electrode are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

### Advantageous Effects

Surprisingly, it was found that the organic electronic device according to the invention solves the problem underlying the present invention by enabling devices in various aspects superior over the organic electroluminescent devices known in the art, in particular with respect to operating voltage over lifetime.

According to one embodiment of the present invention, the substituents on B¹ or R¹ to R⁵ are selected from halogen, with F especially preferred, C₁ to C₃ perhalogenated, especially perfluorinated, alkyl or alkoxy, or -(O)ₗ-CₘH₂ₘ-CₙHalₙ₂ₙ₊₁ with 1= 0 or 1, especially 0, m = 1 or 2, especially 1 and n = 1 to 3, especially 1 or 2 and Hal= halogen, especially F.

According to one embodiment of the present invention, at least one of B¹ or R¹ to R⁵ is substituted alkyl and the substituents of the alkyl moiety are fluorine with the number n_{F} (of fluorine substituents) and n_{H} (of hydrogens) follow the equation: n_{F} > n_{H} + 2.

According to one embodiment of the present invention, at least one of B¹ or R¹ to R⁵ is selected from perfluorinated alkyl or aryl.

According to one embodiment of the present invention, B¹ is substituted C₁ to C₆ alkyl.

According to one embodiment of the present invention, B¹ is substituted C₃ to C₆ linear or cyclic alkyl.

According to one embodiment of the present invention, the compound of formula (1) is free of alkoxy, COR⁶ and/or COOR⁶ groups.

According to one embodiment of the present invention, at least one of R¹ to R⁵ is trifluoromethyl.

According to one embodiment of the present invention, one or two of R¹ to R⁵ are trifluoromethyl.

According to one embodiment of the present invention, one or two of R¹ to R⁵ are trifluoromethyl, and B¹ is substituted C₁ to C₆ alkyl; preferably one or two of R¹ to R⁵ are trifluoromethyl, and B¹ is substituted C₁ to C₄ alkyl.

According to one embodiment of the present invention, one or two of R¹ to R⁵ are trifluoromethyl, and B¹ is perfluorinated C₁ to C₆ alkyl; preferably one or two of R¹ to R⁵ are trifluoromethyl, and B¹ is perfluorinated C₁ to C₄ alkyl.

According to one embodiment of the present invention, at least one of R¹ to R⁵ is trifluoromethyl and the other R¹ to R⁵ are H or F.

According to one embodiment of the present invention, one or two of R¹ to R⁵ are trifluoromethyl and the other R¹ to R⁵ are H or F.

According to one embodiment of the present invention, one or two of R¹ to R⁵ are trifluoromethyl and the other R¹ to R⁵ are H or F, and B¹ is substituted C₁ to C₆ alkyl; preferably one or two of R¹ to R⁵ are trifluoromethyl and the other R¹ to R⁵ are H or F, and B¹ is substituted C₁ to C₄ alkyl.

According to one embodiment of the present invention, one or two of R¹ to R⁵ are trifluoromethyl and the other R¹ to R⁵ are H or F, and B¹ is perfluorinated C₁ to C₆ alkyl; preferably one or two of R¹ to R⁵ are trifluoromethyl and the other R¹ to R⁵ are H or F, and B¹ is perfluorinated C₁ to C₄ alkyl.

According to one embodiment, the anion in compound of formula (1) is selected from the anions A-1 to A-29:

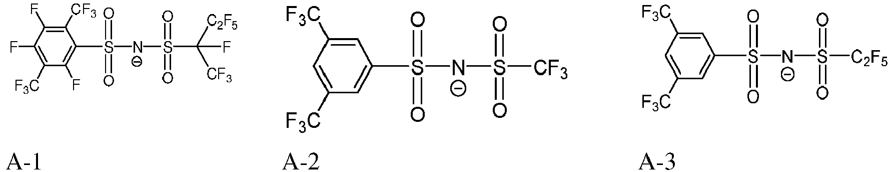

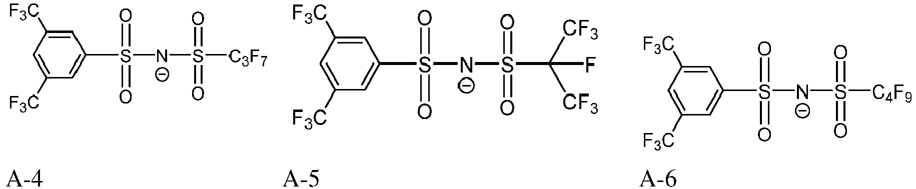

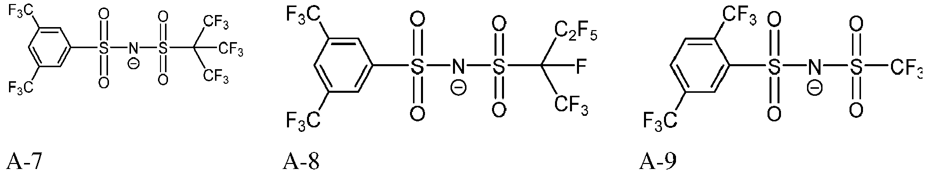

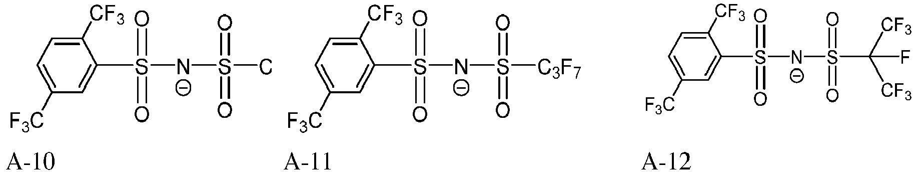

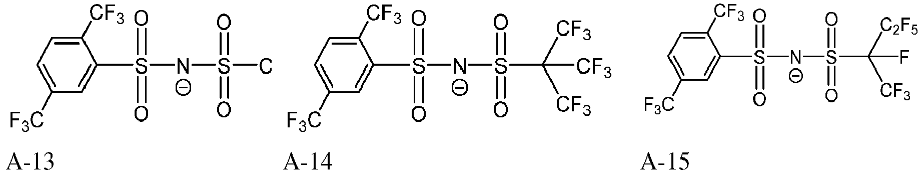

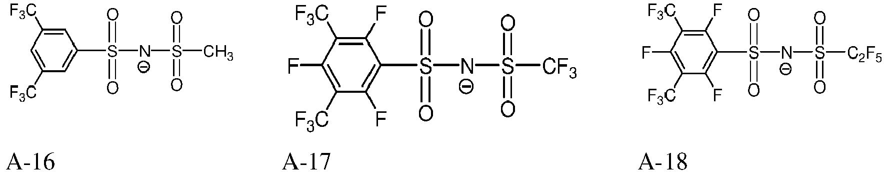

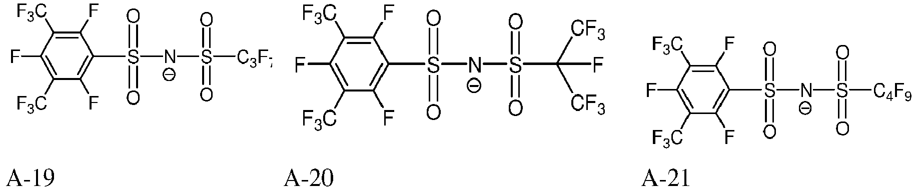

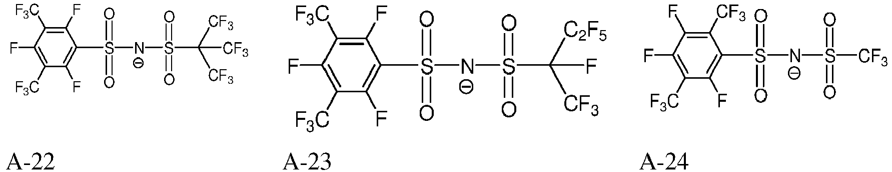

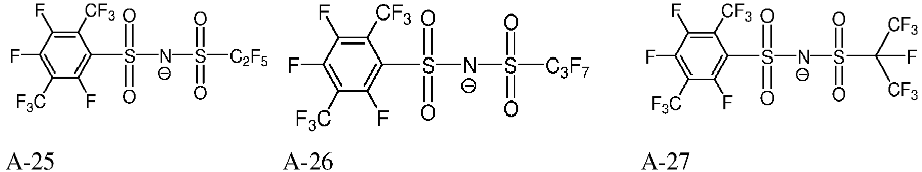

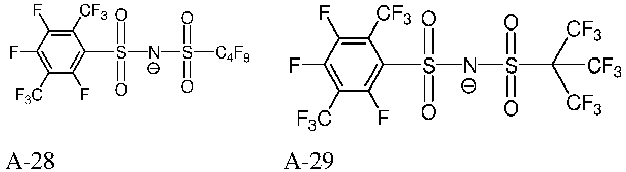

According to one embodiment of the present invention, M has an atomic mass of ≥ 22Da, alternatively ≥ 24Da.

According to one embodiment of the present invention, M is selected from a metal ion wherein the corresponding metal has an electronegativity value according to Allen of less than 2, preferably less than 2, more preferred less than 1.9. Thereby, particularly good performance in organic electronic devices may be achievable.

The term "electronegativity according to Allen" especially refers to Allen, Leland C. (1989). "Electronegativity is the average one-electron energy of the valence-shell electrons in ground-state free atoms". Journal of the American Chemical Society. 111 (25): 9003-9014.

According to one embodiment of the present invention the valency n of M is 1 or 2.

According to one embodiment of the present invention, M is selected from a metal ion wherein the corresponding metal has an electronegativity value according to Allen of less than 2.4, preferably less than 2, more preferred less than 1.9, and the valency n of M is 1 or 2.

According to one embodiment of the present invention, M is selected from an alkali, alkaline earth, rare earth or transition metal, alternatively M is selected from alkali, alkaline earth, or a period 4 or 5 transition metal.

According to one embodiment of the present invention, M is selected from a metal ion wherein the corresponding metal has an electronegativity value according to Allen of less than 2.4, preferably less than 2, more preferred less than 1.9 and M is selected from alkali, alkaline earth, rare earth or a period 4 or 5 transition metal and M has an atomic mass of ≥ 22Da, alternatively ≥ 24Da.

According to one embodiment of the present invention, M is selected from Li, Na, K, Cs, Mg, Mn, Cu, Zn, Ag and Mo; preferably M is selected from Na, K, Cs, Mg, Mn, Cu, Zn and Ag; also preferred M is selected from Na, K, Mg, Mn, Cu, Zn and Ag, wherein if M is Cu, n is 2.

According to one embodiment of the present invention, M is not Ag.

According to one embodiment of the present invention, M is not Cu.

According to one embodiment of the present invention the compound of formula (1) is selected from the compounds A1 to A6:

| **Name** | **Structure** |
|---|---|
| A1 | 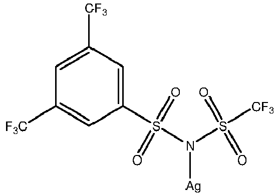 |
| A2 | 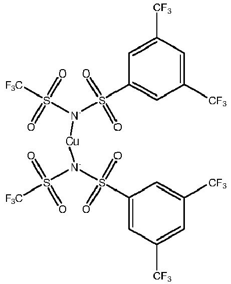 |
| A3 | 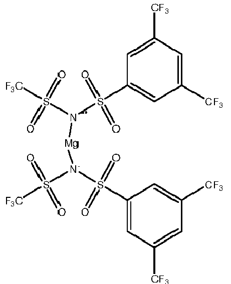 |
| A4 | 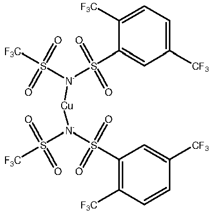 |
| A5 | 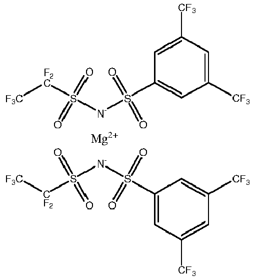 |
| A6 | 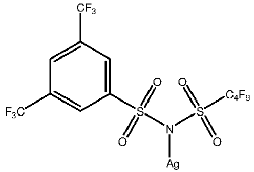 |

According to one embodiment of the present invention the semiconductor layer and/or the compound of formula (1) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the invention, at least one semiconductor layer is arranged and/or provided adjacent to the anode.

According to one embodiment of the invention, at least one semiconductor layer is in direct contact with the anode.

According to one embodiment of the invention, at least one semiconductor layer of the present invention is a hole-injection layer.

In case the at least one semiconductor layer of the present invention is a hole-injection layer and/ or is arranged and/or provided adjacent to the anode then it is especially preferred that this layer consists essentially of the compound of formula (1).

In the context of the present specification the term "consisting essentially of " especially means and/or includes a concentration of ≥ 90% (vol/vol) more preferred ≥ 95% (vol/vol) and most preferred ≥ 99% (vol/vol).

According to another aspect, the at least one semiconductor layer may have a layer thickness of at least about ≥ 0.5 nm to about ≤ 10 nm, preferably of about ≥ 2 nm to about ≤ 8 nm, also preferred of about ≥ 3 nm to about ≤ 5 nm.

According to one embodiment of the invention, at least one semiconductor layer of the present invention further comprises a substantially covalent matrix compound. Preferably at least one semiconductor layer further comprising a substantially covalent matrix compound is arranged and/or provided adjacent to the anode.

Preferred examples of covalent matrix compounds are organic compounds, consisting predominantly from covalently bound C, H, O, N, S, which may optionally comprise also covalently bound B, P, As, Se. Organometallic compounds comprising covalent bonds carbon-metal, metal complexes comprising organic ligands and metal salts of organic acids are further examples of organic compounds that may serve as organic substantially covalent matrix compounds.

In one embodiment, the substantially covalent matrix compound lacks metal atoms and majority of its skeletal atoms is selected from C, O, S, N. Alternatively, the substantially covalent matrix compound lacks metal atoms and majority of its skeletal atoms is selected from C and N.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

In one embodiment, the calculated HOMO level of the substantially covalent matrix compound may be more negative than -4.27 eV, preferably more negative than -4.3 eV, alternatively more negative than -4.5 eV, alternatively more negative than -4.6 eV, alternatively more negative than -4.65 eV.

According to another aspect of the present invention, the semiconductor layer further comprises a substantially covalent matrix compound with an oxidation potential more positive than - 0.2 V and more negative than 1.22 V, when measured by cyclic voltammetry in dichloromethane vs. Fc/Fc+, preferably more positive than - 0.18 V and more negative than 1.12 V. Under these conditions the oxidation potential of spiro-MeO-TAD (CAS 207739-72-8) is - 0.07 V.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) and more positive than the HOMO level of N4,N4'"-di(naphthalen-1-yl)-N4,N4'"-diphenyl-[1,1':4',1":4",1'"-quaterphenyl]-4,4"'-diamine when determined under the same conditions.

In one embodiment of the present invention, the substantially covalent matrix compound may be free of alkoxy groups.

In one embodiment, the calculated HOMO level of the substantially covalent matrix compound may be selected in the range of < -4.27 eV and > -4.84 eV, alternatively in the range of < -4.3 eV and > -4.84 eV, alternatively in the range of < -4.5 eV and > -4.84 eV, alternatively in the range of < -4.5 eV and > -4.84 eV, alternatively in the range of < -4.6 eV and > -4.84 eV.

In one embodiment, the calculated HOMO level of the substantially covalent matrix compound may be selected in the range of < -4.27 eV and > -4.8 eV, alternatively in the range of < -4.3 eV and > -4.8 eV, alternatively in the range of < -4.5 eV and > -4.8 eV, alternatively in the range of < -4.5 eV and > -4.8 eV, alternatively in the range of < -4.6 eV and > -4.8 eV, alternatively in the range of < -4.65 eV and > -4.8 eV.

Preferably, the substantially covalent matrix compound comprises at least one arylamine moiety, alternatively a diarylamine moiety, alternatively a triarylamine moiety.

According to another aspect of the present invention, the at least one semiconductor layer further comprises a compound of formula (2): 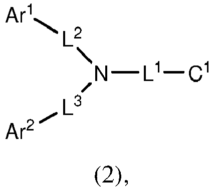 wherein:
L¹ to L³ are independently selected from a single bond, phenylene and naphthenylene, preferably phenylene
Ar¹ and Ar² are independently selected from substituted or unsubstituted C₆ to C₂₀ aryl or substituted or unsubstituted C₃ to C₂₀ heteroarylene;
C¹ is selected from H, an alkyl group which has 1 to 20 carbon atoms and is optionally substituted by one or more R² radicals, or Ar¹;
wherein
R² is the same or different at each instance and is selected from H, D, F, C(-O)R², CN, Si(R³)₃, P(-O)(R³)₂, OR³, S(-O)R³, S(-O)₂R³, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems having 6 to 40 aromatic ring atoms, and heteroaromatic ring systems having 5 to 40 aromatic ring atoms; where two or more R¹ radicals is optionally joined to one another and may form a ring; where the alkyl, alkoxy, alkenyl and alkynyl groups mentioned and the aromatic ring systems and heteroaromatic ring systems mentioned may each be substituted by one or more R³ radicals; and where one or more CH₂ groups in the alkyl, alkoxy, alkenyl and alkynyl groups mentioned is optionally replaced by -R³C-CR³-, -C=C-, Si(R³)₂, C-O, C-NR³, -C(-O)O-, -C(-O)NR³-, P(-O)(R³), -O-, -S-, SO or SO₂;
- the substituents for Ar¹ and Ar² are independently selected from D, C₆ aryl, C₃ to C9 heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN; and
- the substitutents for R³ are independently selected from C₁ to C₆ alkyl, C₆ to C₂₀ aryl and C₅ to C₂₀ heteroaryl, halogen, F or CN.

According to another aspect of the present invention, the at least one semiconductor layer further comprises a compound of formula (2a): 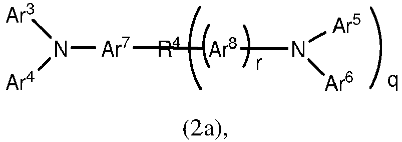 wherein:
Ar⁷ and Ar⁸ are independently selected from substituted or unsubstituted C₆ to C₂₀ arylene or substituted or unsubstituted C₃ to C₂₀ heteroarylene;
Ar³ and Ar⁴ are independently selected from substituted or unsubstituted C₆ to C₂₀ aryl or substituted or unsubstituted C₃ to C₂₀ heteroarylene;
Ar⁵ and Ar⁶ are independently selected from substituted or unsubstituted C₆ to C₂₀ aryl or C₅ to C₄₀ heteroaryl;
R⁴ is a single bond, a unsubstituted or substituted C₁ to C₆ alkyl or phenylene;
   q = 0, 1 or 2;
   r=Oor 1;
   wherein
   - the substituents for Ar³ to Ar⁸ are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN; and
   - the substitutents for R⁴ are independently selected from C₁ to C₆ alkyl, C₆ to C₂₀ aryl and C₅ to C₂₀ heteroaryl, halogen, F or CN.

According to a preferred aspect, the at least semiconductor layer further comprises a compound of formula (2b): 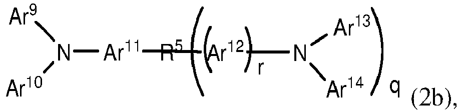 wherein:
Ar⁹ and Ar¹⁰ are independently selected from substituted or unsubstituted C₆ to C₂₀ aryl;
Ar¹¹ and Ar¹² are independently selected from substituted or unsubstituted C₆ to C₂₀ arylene;
Ar¹³ and Ar¹⁴ are independently selected from substituted or unsubstituted C₆ to C₂₀ aryl or C₅ to C₄₀ heteroaryl;
R⁵ is single chemical bond, a unsubstituted or substituted C₁ to C₆ alkyl and unsubstituted or substituted C₁ to C₅ heteroalkyl;
   q = 0, 1 or 2;
   r = 0 or 1;
   wherein
   - the substituents for Ar⁹ to Ar¹⁴ are independently selected from C₁ to C₂₀ alkyl, C₁ to C₂₀ heteroalkyl, or halide; and
   - the substitutents for R⁵ are independently selected from C₁ to C₆ alkyl, C₁ to C₅ heteroalkyl, C₆ to C₂₀ aryl and C₅ to C₂₀ heteroaryl.

According to a further preferred aspect, the semiconductor layer of the present invention may further comprise a compound of formula (2a), wherein Ar¹¹ and Ar¹² are Ph; Ar⁹, Ar¹⁰, Ar¹³ and Ar¹⁴ are selected from phenyl, tolyl, xylyl, mesityl, biphenyl, 1-naphthyl, 2-napthyl, 2-(9,9-dialkyl-fluorenyl), 2-(9-alkyl-9'-aryl-fluorenyl) and 2-(9,9-diaryl-fluorenyl); R⁵ = single bond; r = 1 and q= 1.

According to a further preferred aspect, the semiconductor layer of the present invention may further comprise a compound of formula (2a), wherein Ar¹¹ and Ar¹² are independently selected from phenyl and biphenyl; Ar⁹, Ar¹⁰, Ar¹³ and Ar¹⁴ are selected from phenyl, tolyl, xylyl, mesityl, biphenyl, 1-naphthyl, 2-napthyl, 2-(9,9-dialkyl-fluorenyl), 2-(9-alkyl-9'-aryl-fluorenyl) and 2-(9,9-diaryl-fluorenyl); R⁵ = single bond; r = 1 and q= 1.

According to a further preferred aspect, the semiconductor layer of the present invention may further comprise a compound of formula (2a), wherein Ar¹¹ and Ar¹² are phenyl; Ar⁹, Ar¹⁰, Ar¹³ and Ar¹⁴ are selected from phenyl, tolyl, xylyl, mesityl, biphenyl, 1-naphthyl, 2-napthyl, 2-( 9,9-dialkyl-fluorenyl), 2-(9-alkyl-9'-aryl-fluorenyl) and 2-(9,9-diaryl-fluorenyl); R⁵ = 9,9'-fluorenyl; r = 1 and q= 1.

According to a further preferred aspect, the semiconductor layer of the present invention may further comprise a compound of formula (2a), wherein Ar¹¹ is phenyl; Ar⁹, Ar¹⁰, Ar¹³ and Ar¹⁴ are selected from phenyl, tolyl, xylyl, mesityl, biphenyl, 1-naphthyl, 2-napthyl, 2-(9,9-dialkyl-fluorenyl), 2-(9-alkyl-9'-aryl-fluorenyl) and 2-(9,9-diaryl-fluorenyl); R⁵ = single bond; r = 0 and q= 1. The substituent on Ar¹¹ is selected from phenyl, biphenyl, 2-(9,9-dialkyl-fluorenyl), 2-(9-alkyl-9'-aryl-fluorenyl) and 2-(9,9-diaryl-fluorenyl).

According to a further preferred aspect, the semiconductor layer of the present invention may further comprise a compound of formula (2a), wherein N, Ar⁹ and Ar¹¹ form a carbazole ring; Ar¹² is phenyl or biphenyl; Ar¹⁰, Ar¹³ and Ar¹⁴ are selected from phenyl, tolyl, xylyl, mesityl, biphenyl, 1-naphthyl, 2-napthyl, 2-(9,9-dialkyl-fluorenyl), 2-(9-alkyl-9'-aryl-fluorenyl) and 2-(9,9-diaryl-fluorenyl); R⁵ = single bond; r = 1 and q= 1.

Preferably in Formula (2a) the q may be selected from 1 or 2.

Compounds of formula (2), (2a) or (2b) may have a molecular weight suitable for thermal vacuum deposition. Compounds of formula (2), (2a) or (2b) that can be preferably used as substantially covalent matrix compound may have an molecular weight that is about ≥ 243 g/mol and about ≤ 2000 g/mol, even more preferred is about ≥ 412 g/mol and about ≤ 1800 g/mol, also preferred about ≥ 488 g/mol and about ≤ 1500 g/mol.

According to a more preferred embodiment the Ar¹ and Ar² of Formula (2) may be independently selected from phenylene, biphenylene, naphthylene, anthranylene, carbazolylene, or fluorenylene, preferably from phenylene or biphenylene.

According to a more preferred embodiment the Ar^{x} of Formula (2a) or (2b) may be independently selected from phenyl, biphenyl, terphenyl, quartphenyl, fluorenyl, 9,9'-dimethylfluorenyl, 9,9'-diphenylfluorenyl, 9,9'-spirobi[fluorene]-yl, napthyl, anthranyl, phenanthryl, thiophenyl, fluorenyl, or carbazolyl.

Even more preferred, Ar^{x} of Formula (2a) or (2b) may be independently selected from phenyl, biphenyl, fluorenyl, napthyl, thiopheneyl, fluorenyl, 9,9'-dimethylfluorenyl, 9,9'-diphenylfluorenyl, 9,9'-spirobi[fluorene]-yl, or carbazolyl.

At least two of Ar^{x} of Formula (2a) or (2b) may form a cyclic structure, for example Ar³ and Ar⁴; or Ar³ and Ar⁷; or Ar⁹ and Ar¹⁰; or Ar⁹ and Ar¹¹; may be - wherever possible - a carbazole, phenazoline or phenoxazine ring.

According to a further preferred embodiment the compound has the Formula (2a), wherein:
Ar⁷ and Ar⁷ are independently selected from phenylene, biphenylene, naphthylene, anthranylene, carbazolylene and fluorenylene, preferably selected from phenylene and biphenylene;
Ar³ to Ar⁶ are independently selected from phenyl, biphenyl, terphenyl, quartphenyl, fluorenyl, 9,9'-dimethylfluorenyl, 9,9'-diphenylfluorenyl, 9,9'-spirobi[fluorene]-yl, napthyl, anthranyl, phenanthryl, thiophenyl, 9-carbazolyl; preferably
Ar³ to Ar⁶ are independently selected from phenyl, biphenyl, fluorenyl, 9,9'-dimethylfluorenyl, 9,9'-diphenylfluorenyl, 9,9'-spirobi[fluorene]-yl, napthyl, thiophenyl, carbazolyl.

Furthermore preferred, at least one of Ar³ to Ar⁸ of Formula (2a) may be unsubstituted, even more preferred at least two of Ar³ to Ar⁷ of Formula (2a) may be unsubstituted.

According to an additional preferred embodiment the compound having the Formula (2a):
- Ar³ and Ar⁴ and/or Ar⁵ and Ar⁶ are linked to form a carbazole, phenazoline or phenoxazine ring.

Compounds of formula (2), (2a) or (2b), wherein not all Ar¹ to Ar⁸ are substituted are particularly suited for vacuum thermal deposition.

Preferably, the at least one semiconductor layer further comprises a compound of formula (2a), wherein the substituents on Ar³ to Ar⁶ are independently selected from C₁ to C₁₂ alkyl, C₁ to C₁₂ alkoxy or halide, preferably from C₁ to C₈ alkyl or C₁ to C₈ heteroalkyl, even more preferred from C₁ to C₅ alkyl or C₁ to C₅ heteroalkyl.

Preferably, the at least one semiconductor layer further comprises a compound of formula (2a), wherein the substituents on Ar³ to Ar⁶ are independently selected from C₁ to C₁₂ alkyl or halide, preferably from C₁ to C₈ alkyl or fluoride, even more preferred from C₁ to C₅ alkyl or fluoride.

According to a furthermore preferred embodiment the substantially covalent matrix compound has the Formula (T-1) to (T-6) as shown in Table 1.

**Table 1**

| Name | Chemical formula | Calculated HOMO (eV) |
|---|---|---|
| N,N'-Bis-(3-methylphenyl)-N,N'-bis-(phenyl)-benzidine (T-1) | 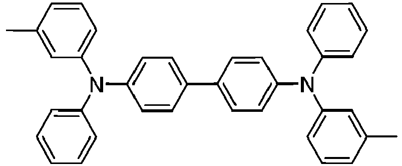 | -4.69 |
| Biphenyl-4-yl(9,9-diphenyl-9H-fluoren-2-yl)-[4-(9-phenyl-9H-carbazol-3-yl)phenyl]-amine (T-2) | 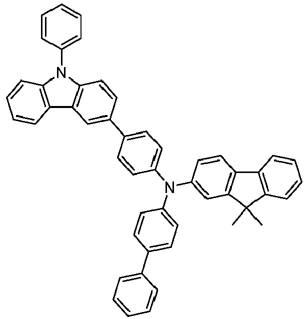 | -4.69 |
| N,N'-Bis(naphthalen-1-yl)-N,N'-bis(phenyl)-benzidine (T-3) | 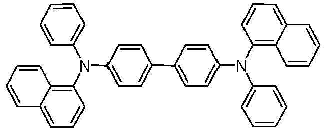 | -4.72 |
| N4,N4,N4',N4'-tetra(biphenyl-4-yl)biphenyl-4,4'-diamine (T-4) | 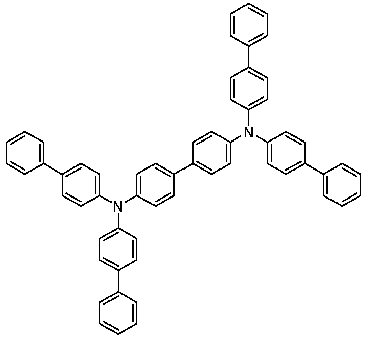 | -4.73 |
| N4,N4"-di(naphthalen-1-yl)-N4,N4"-diphenyl-[1,1':4',1"-terphenyl]-4,4"-diamine (T-5) | 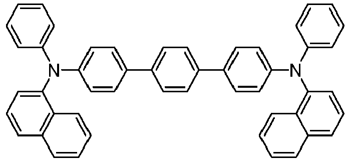 | -4.81 |
| 9,9-dimethyl-N,N-bis(4-(naphthalen- 1 -yl)phenyl)-9H-fluoren-2-amine (T-6) | 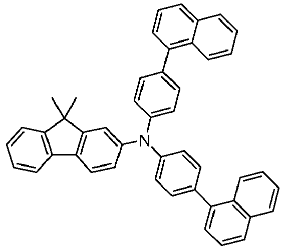 | -4.84 |

According to another aspect, the at least one semiconductor layer further comprises a substantially covalent matrix compound and may comprise:
- at least about ≥ 0.1 wt.-% to about ≤ 50 wt.-%, preferably about ≥ 1 wt.-% to about ≤ 25 wt.-%, and more preferred about ≥ 2 wt.-% to about ≤ 15 wt.-%, of a compound of formula (1), and
- at least about ≥ 50 wt.-% to about ≤ 99 wt.-%, preferably about ≥ 75 wt.-% to about ≤ 99 wt.-%, and more preferred about ≥ 85 wt.-% to about ≤ 98 wt.-%, of a compound of formula (2), (2a) or (2b); preferably the wt.-% of the compound of formula (2), (2a) or (2b) is higher than the wt.-% of the compound of formula (1); wherein the weight-% of the components are based on the total weight of the semiconductor layer.

According to one embodiment of the invention, the at least one semiconductor layer may further comprise a substantially covalent matrix compound and may comprise ≥ 1 and ≤ 30 mol.-% of a compound of formula (1) and ≤ 99 and ≥ 70 mol.-% of a substantially covalent matrix compounds; alternatively ≥ 5 and ≤ 20 mol.-% of a compound of formula (1) and ≤ 95 and ≥ 80 mol.-% of a substantially covalent matrix compounds.

According to one embodiment of the invention the electronic organic device is an electroluminescent device, preferably an organic light emitting diode.

The present invention furthermore relates to a display device comprising an organic electronic device according to the present invention.

The present invention furthermore relates to a compound of formula (1a): 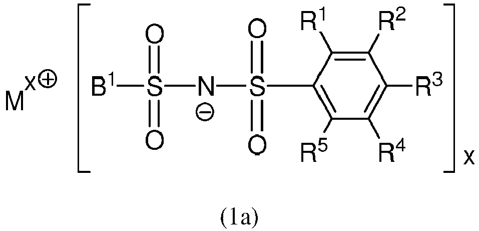 Wherein
M is a metal ion
x is the valency of M
B¹ is selected from substituted or unsubstituted C₁ to C₁₆ alkyl,
R¹ to R⁵ are independently selected from H, F, CN, halogen, substituted or unsubstituted C₁ to C₆ alkyl, substituted or unsubstituted C₆ to C₁₂ aryl, substituted or unsubstituted C₃ to C₁₂ heteroaryl,
wherein the substituents on B¹ and/or R¹ to R⁵ selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR⁶, COOR⁶, halogen, F or CN;
and where at least one of R¹ to R⁵ is selected from substituted or unsubstituted C₁ to C₆ alkyl or CN and
wherein the following compounds are excluded where all of the following is fulfilled:
   M is Li or K;
   x is 1;
   B¹ is CF₃;
   R¹, R³, and R⁵ are H;
   R² and R⁴ are CF₃.

The negative charge in compounds of formula (1) may be delocalised partially or fully over the N(SO₂)₂ group and optionally also over the B¹ and substituted phenyl groups.

Any specifications of formula (1) as described above in the context of the organic electronic device apply *mutatis mutandis.*

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode electrode

The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

### Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

In one embodiment of the present invention, the organic electronic device further comprises a hole transport layer, wherein the hole transport layer is arranged between the semiconductor layer and the at least one photoactive layer.

In one embodiment, the hole transport layer comprises a substantially covalent matrix compound.

In one embodiment of the present invention, the at least one semiconductor layer and the hole transport layer comprise a substantially covalent matrix compound, wherein the substantially covalent matrix compound is selected the same in both layers.

In one embodiment, the hole transport layer comprises a compound of formula (2), (2a) or (2b).

In one embodiment of the present invention, the at least one semiconductor layer and the hole transport layer comprise a compound of formula (2), (2a) or (2b).

In one embodiment of the present invention, the at least one semiconductor layer comprises a compound of formula (1) and a compound of formula (2), (2a) or (2b) and the hole transport layer comprises a compound of formula (2), (2a) or (2b), wherein the compound of formula (2), (2a) or (2b) are selected the same.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime may be improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

It may be provided that the photoactive layer does not comprise the compound of Formula (1).

The photoactive layer may be a light-emitting layer or a light-absorbing layer.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (1).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode electrode

The cathode electrode is formed on the ETL or optional EIL. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

It is to be understood that the cathode electrode is not part of an electron injection layer or the electron transport layer.

### Organic light-emitting diode (OLED)

The organic electronic device according to the invention may be an organic light-emitting device.

According to one aspect of the present invention, there is provided an organic light-emitting diode (OLED) comprising: a substrate; an anode electrode formed on the substrate; an semiconductor layer comprising compound of formula (1), a hole transport layer, an emission layer, an electron transport layer and a cathode electrode.

According to another aspect of the present invention, there is provided an OLED comprising: a substrate; an anode electrode formed on the substrate; a semiconductor layer comprising a compound of Formula (1), a hole transport layer, an electron blocking layer, an emission layer, a hole blocking layer, an electron transport layer and a cathode electrode.

According to another aspect of the present invention, there is provided an OLED comprising: a substrate; an anode electrode formed on the substrate; a semiconductor layer comprising a compound of Formula (1), a hole transport layer, an electron blocking layer, an emission layer, a hole blocking layer, an electron transport layer, an electron injection layer, and a cathode electrode.

According to various embodiments of the present invention, there may be provided OLEDs layers arranged between the above mentioned layers, on the substrate or on the top electrode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adj acent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

The semiconductor layer according to the invention may be the first hole injection layer and p-type charge generation layer.

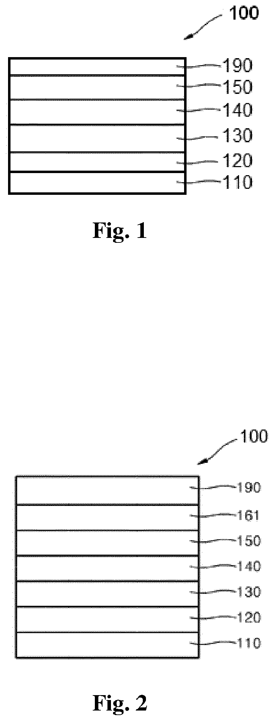

For example, the OLED according to Fig. 2 may be formed by a process, wherein on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

### Organic electronic device

The organic electronic device according to the invention may be a light emitting device, or a photovoltaic cell, and preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound of Formula (1) according to the invention, and
- a second deposition source to release the substantially covalent matrix compound; the method comprising the steps of forming the semiconductor layer; whereby for an organic light-emitting diode (OLED):
- the semiconductor layer is formed by releasing the compound of Formula (1) according to the invention from the first deposition source and the substantially covalent matrix compound from the second deposition source.

According to various embodiments of the present invention, the method may further include forming on the anode electrode, at least one layer selected from the group consisting of forming a hole transport layer or forming a hole blocking layer, and an emission layer between the anode electrode and the first electron transport layer.

According to various embodiments of the present invention, the method may further include the steps for forming an organic light-emitting diode (OLED), wherein
- on a substrate an anode electrode is formed,
- on the anode electrode a semiconductor layer comprising a compound of formula (1) is formed,
- on the semiconductor layer comprising a compound of formula (1) a hole transport layer is formed,
- on the hole transport layer an emission layer is formed,
- on the emission layer an electron transport layer is formed, optionally a hole blocking layer is formed on the emission layer,
- and finally a cathode electrode is formed,
- optional a hole blocking layer is formed in that order between the first anode electrode and the emission layer,
- optional an electron injection layer is formed between the electron transport layer and the cathode electrode.

According to various embodiments, the OLED may have the following layer structure, wherein the layers having the following order:
anode, semiconductor layer comprising a compound of Formula (1) according to the invention, first hole transport layer, second hole transport layer, emission layer, optional hole blocking layer, electron transport layer, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 1 is a schematic sectional view of an organic light-emitting diode (OLED), according to an exemplary embodiment of the present invention;
FIG. 2 is a schematic sectional view of an organic light-emitting diode (OLED), according to an exemplary embodiment of the present invention;
FIG. 3 is a schematic sectional view of an organic light-emitting diode (OLED), according to an exemplary embodiment of the present invention.

FIG. 1 is a schematic sectional view of an organic light-emitting diode (OLED) 100, according to an exemplary embodiment of the present invention. The OLED 100 includes a substrate 110. On the substrate 110 an anode 120 is disposed. On the anode 120 a semiconductor layer comprising a compound of formula (1) is disposed and thereon a hole transport layer 140. Onto the hole transport layer 140 an emission layer 150 and an cathode electrode 190, exactly in this order, are disposed.

FIG. 2 is a schematic sectional view of an organic light-emitting diode (OLED) 100, according to an exemplary embodiment of the present invention. The OLED 100 includes a substrate 110, a first electrode 120, a semiconductor layer comprising a compound of formula (1) 130, a hole transport layer (HTL) 140, an emission layer (EML) 150, an electron transport layer (ETL) 161. The electron transport layer (ETL) 161 is formed directly on the EML 150. Onto the electron transport layer (ETL) 161 a cathode electrode 190 is disposed.

Instead of a single electron transport layer 161, optional an electron transport layer stack (ETL) can be used.

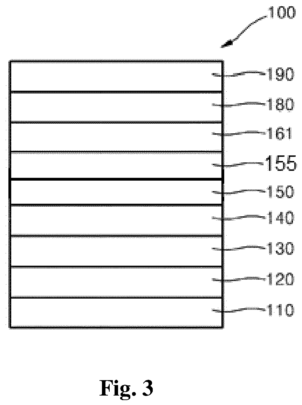

Fig. 3 is a schematic sectional view of an OLED 100, according to another exemplary embodiment of the present invention. Fig. 3 differs from Fig. 2 in that the OLED 100 of Fig. 3 comprises a hole blocking layer (HBL) 155 and an electron injection layer (E1L) 180.

Referring to Fig. 3 the OLED 100 includes a substrate 110, an anode electrode 120, a semiconductor layer comprising a compound of formula (1) 130, a hole transport layer (HTL) 140, an emission layer (EML) 150, a hole blocking layer (HBL) 155, an electron transport layer (ETL) 161, an electron injection layer (EIL) 180 and a cathode electrode 190. The layers are disposed exactly in the order as mentioned before.

In the description above the method of manufacture an OLED of the present invention is started with a substrate 110 onto which an anode electrode 120 is formed, on the anode electrode 120, an hole injection layer 130, hole transport layer 140, an emission layer 150, optional a hole blocking layer 155, optional at least one electron transport layer 161, optional at least one electron injection layer 180, and a cathode electrode 190 are formed, exactly in that order or exactly the other way around.

While not shown in Fig. 1, Fig. 2 and Fig. 3, a sealing layer may further be formed on the cathode electrodes 190, in order to seal the OLEDs 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Detailed description

The invention is furthermore illustrated by the following examples which are illustrative only and non-binding.

The compounds of the present invention can be made by methods known to the skilled person in the art, some general procedures with exemplified educts are described in the following:

### General procedure for synthesis of sulfonamide ligand

3,5-bis(trifluoromethyl)sulfonylchloride was dissolved in dry acetone (ca. 10ml/g) and 3eq of K₂CO₃ was added. The mixture was cooled in an ice bath. 1eq of the desired B¹ sulfonamide was added in counter flow. The mixture was stirred at room temperature, until ¹⁹F-NMR shows complete conversion. The solid was filtered off and washed with acetone. The solvent was removed under reduced pressure. The residue was treated with ice-cold half concentrated sulfuric acid and extracted with diethyl ether. The combined organic layers were washed with a small amount of water, dried over sodium sulfate and the solvent removed under reduced pressure. The residue was distilled from bulb to bulb in high vacuum.

### General procedure for compounds of formula (I) wherein M is Cu(II)

The sulfonamide ligand was dissolved in water (ca 10ml/g) and 0.5 eq Cu(OAc)₂ was added. The mixture was stirred until a clear blue solution was obtained. The solvent was removed under reduced pressure. Residual acetic acid was removed by repeated adding of toluene and removal of solvents under reduced pressure. The crude material was purified by sublimation.

### General procedure for compounds of formula (I) wherein M is Mn(II)

The sulfonamide ligand was dissolved in MeOH (ca. 10ml/g) and carefully securated by bubbling nitrogen through the vigorously stirred solution. 0.5 eq metallic Mn powder was added and the mixture was stirred overnight at room temperature. The solvent was removed under reduced pressure and the remaining oil was stirred in degassed water to obtain a solid. The crude material was purified by sublimation.

### General procedure for compounds of formula (I) wherein M is Mg(II)

The sulfonamide ligand was suspended under inert conditions in dry toluene (ca. 5ml/g) and dissolved at 50°C. 0.5 eq. MgBu₂ solution in heptane was added dropwise. The reaction mixture was stirred at 50°C for 2h. After cooling, the product was precipitated with dry hexane (ca. 10ml/g). The precipitate was filtered off under inert conditions, washed with dry hexane and dried in high vacuum. The crude product was purified by sublimation.

As comparative examples, the following compounds were used:

| **Comparative example No** | **Structure** |
|---|---|
| 1 | Cu (TFSI)₂ |
| 2 | Ag (TFSI) |
| 3 | Li (TFSI) |

### Sublimation temperature

Under nitrogen in a glovebox, 0.5 to 5 g compound are loaded into the evaporation source of a sublimation apparatus. The sublimation apparatus consist of an inner glass tube consisting of bulbs with a diameter of 3 cm which are placed inside a glass tube with a diameter of 3.5 cm. The sublimation apparatus is placed inside a tube oven (Creaphys DSU 05/2.1). The sublimation apparatus is evacuated via a membrane pump (Pfeiffer Vacuum MVP 055- 3C) and a turbo pump (Pfeiffer Vacuum THM071 YP). The pressure is measured between the sublimation apparatus and the turbo pump using a pressure gauge (Pfeiffer Vacuum PKR 251). When the pressure has been reduced to 10⁻⁵ mbar, the temperature is increased in increments of 10 to 30 K till the compound starts to be deposited in the harvesting zone of the sublimation apparatus. The temperature is further increased in increments of 10 to 30 K till a sublimation rate is achieved where the compound in the source is visibly depleted over 30 min to 1 hour and a substantial amount of compound has accumulated in the harvesting zone.

The sublimation temperature, also named T_{subl}, is the temperature inside the sublimation apparatus at which the compound is deposited in the harvesting zone at a visible rate and is measured in degree Celsius.

In the context of the present invention, the term "sublimation " may refer to a phase transfer from solid state to gas phase or from liquid state to gas phase.

### Decomposition temperature

The decomposition temperature, also named T_{dec}, is determined in degree Celsius.

The decomposition temperature is measured by loading a sample of 9 to 11 mg into a Mettler Toledo 100 µL aluminum pan without lid under nitrogen in a Mettler Toledo TGA-DSC 1machine. The following heating program was used: 25°C isothermal for 3 min; 25°C to 600°C with 10 K/min.

The decomposition temperature was determined based on the onset of the decomposition in TGA.

### Rate onset temperature

The rate onset temperature (T_{RO}) is determined by loading 100 mg compound into a VTE source. As VTE source a point source for organic materials may be used as supplied by Kurt J. Lesker Com-pany (www.lesker.com) or CreaPhys GmbH (http://www.creaphys.com). The VTE source is heated at a constant rate of 15 K/min at a pressure of less than 10⁻⁵ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

### Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard Fc⁺/Fc redox couple, afforded finally the values reported above. All studied compounds as well as the reported comparative compounds showed well-defined reversible electrochemical behaviour.

### Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected. The HOMO and LUMO levels are recorded in electron volt (eV).

### General procedure for fabrication of OLEDs

For OLEDs, see Examples 5 and 6 and comparative example 3 in Table 3, a 15Ω/cm² glass substrate with 90 nm ITO (available from Corning Co.) was cut to a size of 50 mm x 50 mm x 0.7 mm, ultrasonically washed with isopropyl alcohol for 5 minutes and then with pure water for 5 minutes, and washed again with UV ozone for 30 minutes, to prepare the anode.

Then, 92 mol.-% Biphenyl-4-yl(9,9-diphenyl-9H-fluoren-2-yl)-[4-(9-phenyl-9H-carbazol-3-yl)phenyl]-amine (CAS 1242056-42-3) with 8 mol.-% compound of formula (1) was vacuum deposited on the anode, to form a HIL having a thickness of 10 nm. In comparative examples 4 and 5, the compounds shown in Table 3 were used in place of compounds of formula (1).

Then, Biphenyl-4-yl(9,9-diphenyl-9H-fluoren-2-yl)-[4-(9-phenyl-9H-carbazol-3-yl) phenyl]-amine was vacuum deposited on the HIL, to form a first HTL having a thickness of 128 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a first blue-emitting emission layer (EML) with a thickness of 20 nm.

Then a hole blocking layer is formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer.

Then, the electron transporting layer having a thickness of 31 nm is formed on the hole blocking layer by depositing 4'-(4-(4-(4,6-diphenyl-1,3,5-triazin-2-yl)phenyl)naphthalen-1-yl)-[1,1'-biphenyl]-4-carbonitrile and LiQ in a ratio of 50:50 vol.-%.

A1 is evaporated at a rate of 0.01 to 1 Å/s at 10⁻⁷ mbar to form a cathode with a thickness of 100 nm.

A cap layer of Biphenyl-4-yl(9,9-diphenyl-9H-fluoren-2-yl)-[4-(9-phenyl-9H-carbazol-3-yl)phenyl]-amine is formed on the cathode with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm² is determined by interpolating the luminance-voltage and current-voltage characteristics, respectively.

Lifetime LT of the device is measured at ambient conditions (20°C) and 30 mA/cm², using a Keithley 2400 sourcemeter, and recorded in hours.

The brightness of the device is measured using a calibrated photo diode. The lifetime LT is defined as the time till the brightness of the device is reduced to 97 % of its initial value.

To determine the voltage stability over time U(100h)-(1h) and U(100h-50h), a current density of at 30 mA/cm² was applied to the device. The operating voltage was measured after 1 hour, after 50 hours and after 100 hours, followed by calculation of the voltage stability for the time period of 1 hour to 100 hours and for a time period of 50 hours to 100 hours.

### Technical Effect of the invention

In order to investigate the usefulness of the inventive compound preferred materials were tested in view of their thermal properties

As materials for organic electronics are typically purified by sublimation, a large offset between decomposition and sublimation temperature T_{dec}-T_{subl} are highly desirable. Thereby, a high sublimation rate may be achievable.

**Table 2: Properties of compounds of formula (1) and comparative examples 1 and 2:**

| | Name | T_{dec} [°C] | T_{dec}-T_{subl} [°C] |
|---|---|---|---|
| Comparative example 1 | Cu (TFSI)₂ | 180 | 10 |
| Comparative example 2 | Ag (TFSI) | 320 | 5-10°C |
| Example 1 | A1 | > 350 | > 41 |
| Example 2 | A2 | ≥ 265 | ≥ 30 |
| Example 3 | A3 | > 350 | > 41 |
| Example 4 | A5 | > 350 | ≥ 30 |

In Table 2 are shown the temperature at which thermal decomposition is observed (T_{dec}), difference between decomposition and sublimation temperature and yield after purification through sublimation.

The decomposition temperature of Cu (TFSI)₂ is 180 °C, see comparative example 1 in Table 2. The difference between decomposition and sublimation temperature is 10 °C. A sublimation rate which is suitable for mass production cannot be achieved as a substantial amount of compound decomposes before it sublimes.

The decomposition temperature of Ag (TFSI) is 320 °C, see comparative example 2 in Table 2. Comparative example 2 differs from comparative example 1 in the metal ion (Ag⁺ instead of Cu²⁺). The decomposition temperature is increased from 180 °C in comparative example 1 to > 320 °C. The difference between decomposition and sublimation temperature is 5 to 10 °C. Therefore, a high rate in sublimation cannot be achieved easily without decomposition.

Surprisingly, for compounds of formula (1) the temperature difference between decomposition and sublimation temperature is at least 30 °C, see examples 1 to 4 in Table 2.

As materials for organic electronics are typically purified by sublimation, a high decomposition temperature, a large offset between decomposition and sublimation temperature is highly desirable. Thereby, a high sublimation rate may be achievable.

In Table 3 are shown the properties of organic electronic devices comprising compounds of formula (1) and comparative example 3.

**Table 3: Properties of organic electronic device comprising compound of formula 1 and comparative examples 4 and 5**

| | Chemical structure of the compound contained in the device | U(100h)-(1h) @ 30mA/cm² [V] | U(100h)-(50h) @ 30mA/cm² [V] |
|---|---|---|---|
| Comparative example 3 | Li (TFSI) | 1.11 | 0.33 |
| Example 5 | A2 | 0.04 | 0.02 |
| Example 6 | A5 | 0.06 | 0.04 |

A current density of 30 mA/cm² was applied to the devices for 1 hour to achieve stable performance. Then, the change in operating voltage over 100 hours was determined.

In comparative example 3, Li (TFSI) was used. The operating voltage increases by 1.11 V over 100 hours.

Surprisingly, it was found that in devices comprising a compound of formula (1), the operating voltage increases much less over time compared to the comparative examples.

The beneficial effect is even more pronounced when the voltage change between 50 and 100 hours is measured. In comparative example 3, the operating voltage increases by 0.33 V.

In examples comprising compound of formula (I), the operating voltage increases only by 0.02 and 0.04 V, respectively.

A low increase or even decrease in operating voltage over time is highly desirable, as the power consumption over time does not increase. Low power consumption is important for long battery life, in particular in mobile devices.

Thereby, an improvement in performance has been achieved even for stronger oxidizing metal complexes. Without being bound by theory, it is believed that stronger oxidizing metal complexes may enable more effective hole injection into an organic electronic device. Therefore, it is highly desirable to provide stronger oxidizing metal complexes in a form which is suitable for mass production of organic electronic devices.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. An organic electronic device comprising an anode, a cathode, at least one photoactive layer and at least one semiconductor layer, wherein the at least one semiconductor layer is arranged between the anode and the at least one photoactive layer; and wherein the at least one semiconductor layer comprises a compound of Formula (1): 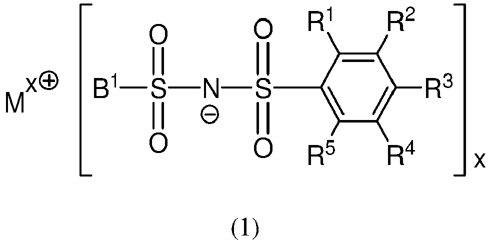 Wherein
M is a metal ion
x is the valency of M
B¹ is selected from substituted or unsubstituted C₁ to C₁₆ alkyl,
R¹ to R⁵ are independently selected from H, F, CN, halogen, substituted or unsubstituted C₁ to C₆ alkyl, substituted or unsubstituted C₆ to C₁₂ aryl, substituted or unsubstituted C₃ to C₁₂ heteroaryl,
wherein the substituents on B¹ and/or R¹ to R⁵ selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR⁶, COOR⁶, halogen, F or CN; and where at least one of R¹ to R⁵ is selected from substituted or unsubstituted C₁ to C₆ alkyl or CN.

2. The organic electronic device of Claim 1 whereby the substituents on B¹ or R¹ to R⁵ are selected from halogen, with F especially preferred, C₁ to C₃ perhalogenated, especially perfluorinated, alkyl or alkoxy, or -(O)ₗ-CₘH₂ₘ-CₙHalₙ₂ₙ₊₁ with l= 0 or 1, especially 0, m = 1 or 2, especially 1 and n = 1 to 3, especially 1 or 2 and Hal= halogen, especially F.

3. The organic electronic device of Claim 1 or 2, whereby at least one of B¹ or R¹ to R⁵ is substituted alkyl and the substituents of the alkyl moiety are fluorine with the number n_{F} (of fluorine substituents) and n_{H} (of hydrogens) follow the equation: n_{F} > n_{H} + 2.

4. The organic electronic device of Claim 1 or 3 whereby at least one of B¹ or R¹ to R⁵ perfluorinated alkyl.

5. The organic electronic device of Claim 1 or 4 whereby at least one of R¹ to R⁵ is trifluoromethyl.

6. The organic electronic device of any of the Claims 1 to 5, whereby M has an atomic mass of ≥ 22 Da.

7. The organic electronic device of any of the Claims 1 to 6, whereby M is selected from a metal ion wherein the corresponding metal has an electronegativity value according to Allen of less than 2.4.

8. The organic electronic device of any of the Claims 1 to 7, whereby the compound of formula (1) is free of alkoxy, COR⁶ and/or COOR⁶ groups.

9. The organic electronic device of any of the claims 1 to 8, whereby the anion of compound (1) is selected from A-1 to A-29:

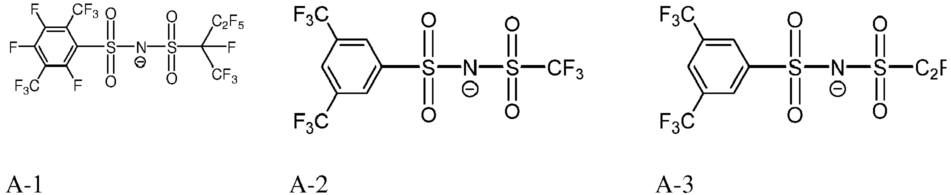

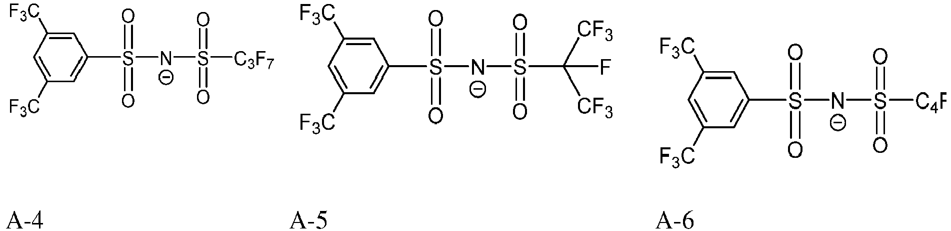

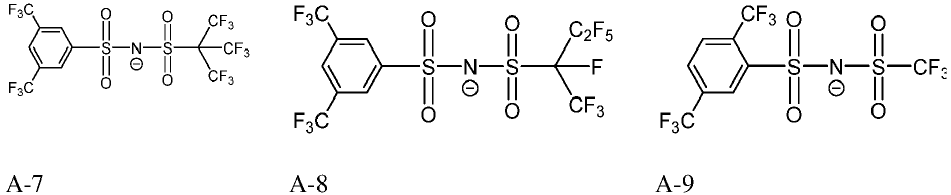

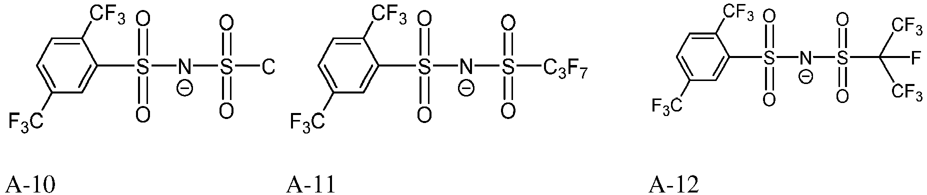

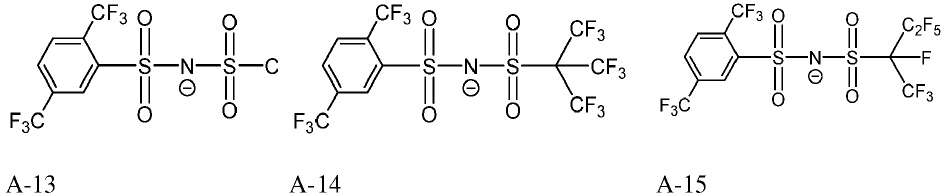

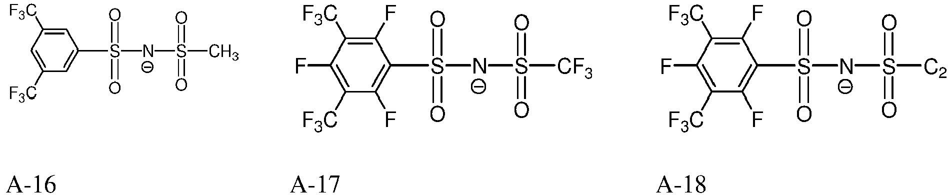

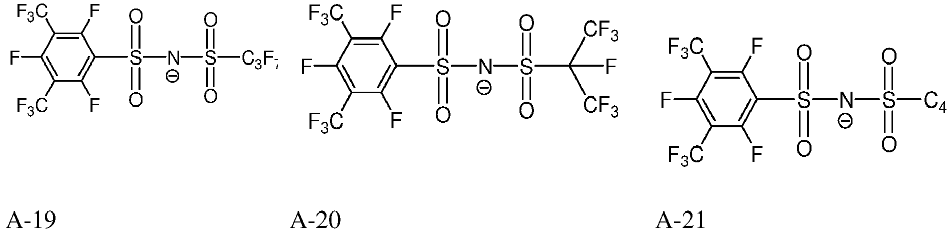

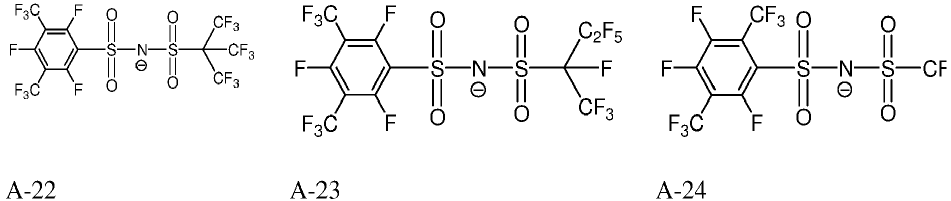

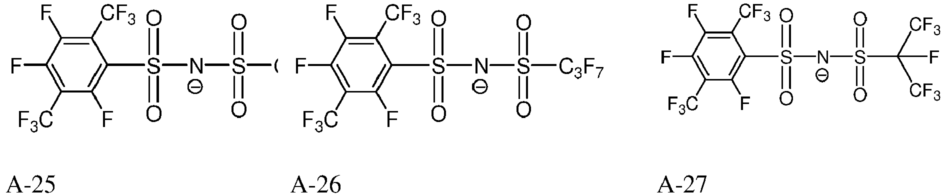

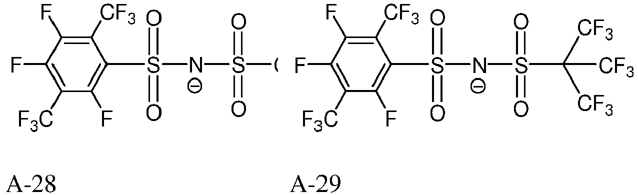

10. The organic electronic device of any of the claims 1 to 9, whereby the at least one semiconductor layer is non-emissive.

11. The organic electronic device of any of the claims 1 to 10, whereby at least one of the semiconductor layers is a hole-injection layer, which consists essentially of the compound of formula (1).

12. The organic electronic device of any of the claims 1 to 11, whereby at least one of the at least one semiconductor layers further comprises a substantially covalent matrix compound.

13. The electronic organic device of any of the claims 1 to 12, whereby the electronic organic device is an electroluminescent device, preferably an organic light emitting diode.

14. A display device comprising an organic electronic device according to any of the claims 1 to 13.

15. A compound of formula (1a): 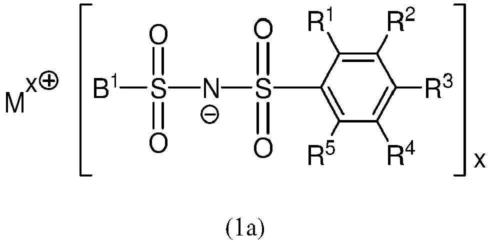 Wherein
M is a metal ion
x is the valency of M
B¹ is selected from substituted or unsubstituted C₁ to C₁₆ alkyl,
R¹ to R⁵ are independently selected from H, F, CN, halogen, substituted or unsubstituted C₁ to C₆ alkyl, substituted or unsubstituted C₆ to C₁₂ aryl, substituted or unsubstituted C₃ to C₁₂ heteroaryl,
wherein the substituents on B¹ and/or R¹ to R⁵ selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR⁶, COOR⁶, halogen, F or CN;
and where at least one of R¹ to R⁵ is selected from substituted or unsubstituted C₁ to C₆ alkyl or CN and
wherein the following compounds are excluded where all of the following is fulfilled:
M is Li or K;
x is 1;
B¹ is CF₃;
R¹, R³, and R⁵ are H;
R² and R⁴ are CF₃.
